# EUROPÄISCHE PATENTSCHRIFT

(11) **EP 0 618 444 B1**
(45) Veröffentlichungstag und Bekanntmachung des Hinweises auf die Patenterteilung: **12.02.1997**
(21) Anmeldenummer: 94100151.3
(22) Anmeldetag: 07.01.1994
(51) Int. Cl.: G01N 21/90, G01F 23/28

(54) **Verfahren und Vorrichtung zum Ermitteln von Restflüssigkeit in einer Flasche**
Method and apparatus for detecting residual liquid in a bottle
Procédé et appareil pour détecter des restes de liquide dans une bouteille

(30) Priorität: 04.03.1993 CH 657/93
(43) Veröffentlichungstag der Anmeldung: 05.10.1994
(73) Patentinhaber: ELPATRONIC AG, CH-6303 Zug (CH)
(72) Erfinder: Gernet, Pierre, CH-5400 Ennetbaden (CH); Alvarez, Fernando, CH-5415 Rieden/Baden (CH)

(56) Entgegenhaltungen:
- EP-A- 0 061 797
- DE-A- 2 738 571
- DE-A- 3 245 908
- FR-A- 2 218 952
- US-A- 3 827 812
- PATENT ABSTRACTS OF JAPAN vol. 7, no. 101 (P-194) (1246) 28. April 1983 & JP-A-58 024 841 (KIRIN BEER K.K.) 14. Februar 1983

## Beschreibung

Die Erfindung betrifft ein Verfahren zum Ermitteln von Restflüssigkeit in einer rücklaufenden Mehrwegflasche, insbesondere in einer PET-Flasche, gemäss Oberbegriff des Anspruchs 1. Ferner betrifft die Erfindung eine Vorrichtung zur Durchführung des Verfahrens gemäss Oberbegriff des Anspruchs 3.

Insbesondere bei Mehrweg-Getränkeflaschen (z.B. bei PET-Flaschen) ist es üblich, am Anfang der Prüfung der rücklaufenden Flaschen eine Prüfung auf das Vorhandensein von Restflüssigkeit in der Flasche durchzuführen. Dabei soll vor allem verhindert werden, dass bei einem späteren Prüfschritt, bei welchem ein Gasentnahmeelement in die Flasche eintaucht, eine Verunreinigung desselben durch die Restflüssigkeit erfolgt. Entsprechend werden Flaschen, welche eine zu grosse Menge Restflüssigkeit enthalten, aufgrund der Prüfung auf das Vorhandensein von Restflüssigkeit aus dem Mehrwegumlauf ausgeschieden, bevor solche Flaschen in die weiteren Prüfstationen gelangen. Ein Verfahren zur Erkennung von Restflüssigkeit sollte daher die zuverlässige Detektion von allen möglichen Stoffen in den Flaschen, z.B. auch in Form von Eis, ermöglichen. Kleine Mengen an Restflüssigkeiten von z.B. 1/3 Liter bis 1/2 Liter sind dabei nicht problematisch und können bei der Prüfung noch als zulässig akzeptiert werden. Das Erkennungsverfahren sollte aber grundsätzlich so empfindlich sein, dass auch kleinere Mengen von Restflüssigkeit erkannt werden können, wenn dies erwünscht ist.

Bekannte Prüfverfahren zur Erkennung von Restflüssigkeit machen von einem Hochfrequenzfeld im Megahertzbereich Gebrauch, welches von der Flasche durchlaufen wird. Der hauptsächliche Nachteil dieses Verfahrens besteht darin, dass keine öligen Flüssigkeiten erkannt werden können. Weiter ist es bekannt, Restflüssigkeit mittels eines Ultraschallsensors festzustellen. Nachteilig an diesem Verfahren ist, dass die Bewegung der Oberfläche der Flüssigkeit in der Flasche, was bei der Flaschenförderung häufig der Fall ist, zu einer mehrfachen Reflektion an der Flüssigkeitsoberfläche und zu nur schwer klar interpretierbaren Messresultaten führt. Bekannt ist es ferner, Erkennungsverfahren mit radioaktiven Quellen oder durch den Einsatz von Mikrowellen auszuführen. Diese Verfahren sind aber in der Praxis mit relativ hohen Kosten der entsprechenden Vorrichtungen verbunden. Weiter ist es bekannt, einen kapazitiven Sensor einzusetzen. Dabei ergibt sich als hauptsächlicher Nachteil, das nasse Papieretiketten an den Flaschen zu einem Ansprechen des Sensors führen können, obwohl keine oder nur unbedenklich wenig Restflüssigkeit in der Flasche vorhanden ist. Weiter ist es bekannt, Restflüssigkeit durch einen Wägevorgang zu erkennen. Auch in diesem Fall ist die apparative Lösung (Waage) relativ teuer.

Aus DE-A-3 245 908 ist ein Vefahren gemäss Oberbegriff bekannt, bei dem Licht durch die Flaschenöffnung eingestrahlt und unterhalb des Flaschenbodens empfangen wird. Der Lichtempfänger kann dabei durch am Flaschenboden haftendes Waschwasser gestört werden und verkratzte Flaschenböden können die Messung beeinflussen.

Der Erfindung liegt deshalb die Aufgabe zugrunde, ein Verfahren zur Ermittlung von Restflüssigkeit zu schaffen, welches rasch und kostengünstig alle Arten von Rückständen erfassen kann, insbesondere auch ölige Rückstände oder auch feste Rückstände oder flüssige Rückstände in fester Form und welches die genannten Nachteile nicht aufweist.

Dies wird erfindungsgemäss durch die kennzeichnenden Merkmale des Anspruchs 1 erreicht.

Es hat sich gezeigt, dass durch die Verwendung von Licht auf die beanspruchte Art, insbesondere im Infrarot-Bereich, zuverlässig das Vorhandensein von Reststoffen in der Flasche erfasst werden kann. Dabei spielt die Art der Stoffe praktisch keine Rolle. Das Verfahren hat sich als ausserordentlich zuverlässig und kostengünstig erwiesen.

Der Erfindung liegt ferner die Aufgabe zugrunde, eine Vorrichtung zur Durchführung des Verfahrens zu schaffen. Dies wird mit den Merkmalen des Anspruchs 3 erreicht.

Im folgenden werden Ausführungsbeispiele der Erfindung anhand der Zeichnungen näher erläutert. Dabei zeigt
Figur 1 schematisch eine im Mehrwegumlauf befindliche Flasche und die Sende- und Empfangseinrichtung zur Durchführung des Verfahrens; und
Figur 2 ein Diagram des Ausgangssignals der Empfangseinheit.

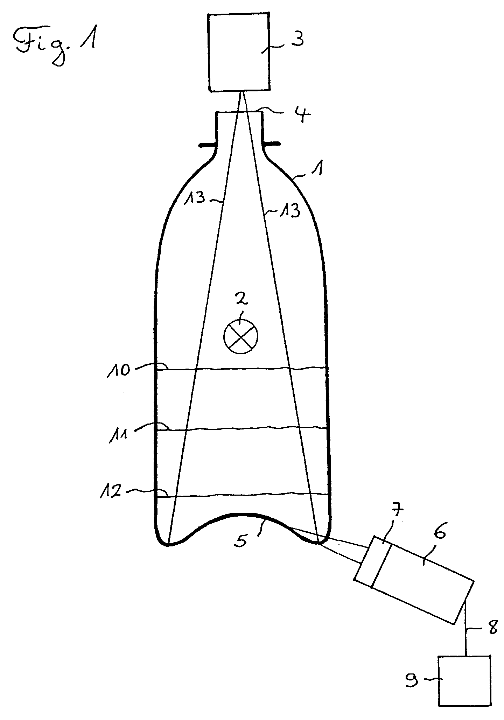

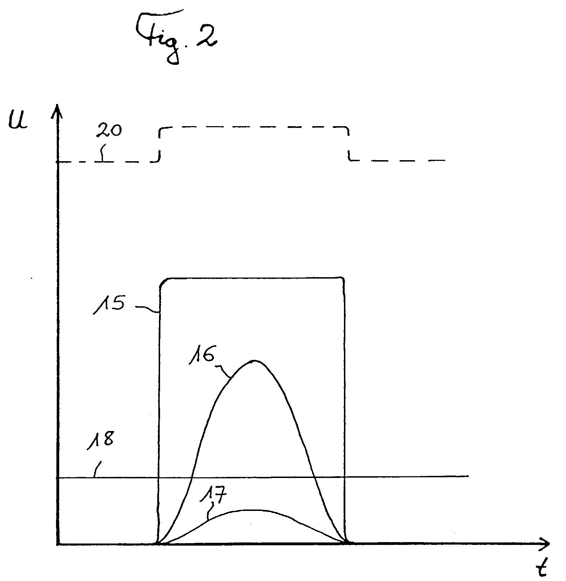

Figur 1 zeigt schematisch eine PET-Flasche 1, welche entlang einer nicht dargestellten Förderstrecke einer Flascheninspektionsmaschine gefördert wird. Die Förderrichtung verläuft dabei z.B. aus der Zeichnungsebene nach hinten, wie durch das Symbol 2 dargestellt. Bei dieser Bewegung gelangt die Flasche in den Sendebereich eines Infrarot-Senders 3. Dieser handelsübliche Sender strahlt Licht im Infrarotbereich ab, welches z.B. durch eine oder mehrere Infrarotdioden erzeugt wird. Anstelle des Infrarot-Senders könnte auch eine Lichtquelle verwendet werden, die im sichtbaren Bereich Licht abstrahlt, doch werden im Infrarot-Bereich bessere Ergebnisse erzielt. Bei der gezeigten Anordnung von Figur 1 erfolgt die Einstrahlung des Infrarot-Lichtes in die Flasche 1 durch die Flaschenöffnung 4 hindurch. Das Infrarot-Licht verläuft innerhalb der Flasche z.B. so, wie es durch den Strahlenkegel 13 angedeutet ist. Natürlich kann durch eine Optik im Sender 3 eine andere Bündelung des Strahls erreicht werden. Bei einer leeren Flasche ohne Restflüssigkeit wird die Intensität des Lichtes in der Flasche praktisch nicht gedämpft. Das die Flasche durchlaufende Licht wird wie in Figur 1 gezeigt, durch einen seitlich des Flaschenbodens angeordneten Empfänger 6 aufgefangen. Aufgefangen wird dabei in der gezeigten Anordnung Licht, welches an der Wölbung 5 des Flaschenbodens reflektiert wird und seitlich aus der Flasche austritt. Am Empfänger 6 kann eine Fokussieroptik 7 vorgesehen sein, welche das austretende Licht auf die Empfangselemente, in der Regel Fotodioden oder Fototransistoren, fokussiert. Im Empfänger 6 wird das empfangene Licht in ein entsprechendes elektrisches Signal umgewandelt, welches über die Signalleitung 8 an eine Auswerteinrichtung 9 abgegeben wird. In der Auswerteinrichtung 9 wird aufgrund des elektrischen Signals erkannt, ob Restflüssigkeit in der Flasche vorhanden ist oder nicht. Durch das Vorhandensein von Restflüssigkeit ergibt sich nämlich eine mehr oder weniger starke Dämpfung des Lichtes in der Flasche, was durch den Empfänger 6 feststellbar ist. Die Dämpfung ist dabei je nach Art der Flüssigkeit verschieden. So kann z.B. ein Pegel 10 einer klaren Flüssigkeit, z.B. Wasser, ungefähr dieselbe Dämpfung ergeben, wie ein Pegel 11 einer trüben Flüssigkeit, z.B. einer Limonade oder ein Pegel 12 einer öligen Flüssigkeit. In Figur 2 ist dargestellt, wie sich das Ausgangssignal des Empfängers 6, wie es mit der Signalleitung 8 weitergegeben wird, beim Durchlauf der Flasche durch die Sende-Empfangsanordnung darstellen kann. Dabei ist in Figur 2 oben mit der gestrichelten Linie 20 der zeitliche Ablauf des Flaschendurchlaufes eingezeichnet. Eine leere Flasche bewirkt dabei ungefähr ein Ausgangssignal, wie es mit der Kurve 15 dargestellt ist. Dies entspricht dem im wesentlichen ungedämpften Verlauf des Lichtes in der Flasche 1. Die Kurve 16 zeigt ungefähr den Verlauf des Ausgangssignals, wie er sich mit Restflüssigkeit in der Flasche bzw. der entsprechenden Dämpfung des Lichtes in der Flasche ergibt. Der Verlauf der Kurve 16 ergibt sich dabei, wenn nur eine relativ geringe, noch akzeptable Menge von Restflüssigkeit in der Flasche vorhanden ist. Eine starke Dämpfung, und entsprechend ein Kurvenverlauf ungefähr gemäss Kurve 17, ergibt sich, wenn viel Flüssigkeit oder eine geringere, aber nicht mehr akzeptable Menge einer öligen, stark dämpfenden Flüssigkeit in der Flasche vorhanden ist. Der Kurvenverlauf gemäss der Kurve 17 kann dabei ungefähr den in Figur 1 gezeigten Pegeln 10, 11,12 entsprechen. In der Auswerteeinrichtung 9 wird nun ein Grenzwert festgelegt, unterhalb dessen die Flasche als zuviel Restflüssigkeit enthaltend vorgemerkt wird. Ein solcher Grenzwert kann z.B. so eingestellt werden, wie dies mit der Linie 18 in Figur 3 angedeutet ist. Flaschen, die ein Signal oberhalb dieses Grenzwerts bewirken, also im gezeigten Beispiel Flaschen, die ein Ausgangssignal gemäss den Kurven 16 oder 15 bewirken, werden aufgrund eines Ausgangssignals aus der Auswerteinrichtung 9 weiter durch die Fördereinrichtung zur nächsten Prüfstation gefördert. Flaschen hingegen, die ein Ausgangssignal des Empfängers 6 unterhalb des Grenzwertes 18 bewirken, z.B. also eine Flasche, welche ein Ausgangssignal gemäss der Kurve 17 bewirkt, werden durch das entsprechende Ausgangssignal der Auswerteinrichtung 9 aus dem Förderweg entfernt. Dies kann auf bekannte Weise z.B. durch einen Auswerfer erfolgen.

Neben Restflüssigkeiten erkennt das beschriebene Verfahren bzw. die Vorrichtung auch stark verschmutzte Flaschen (z.B. Verunreinigung durch Farben) und Feststoffe in den Flaschen. Auch das Entfernen solcher Flaschen aus dem Umlauf ist erwünscht und das erfindungsgemässe Verfahren bietet somit einen Vorteil gegenüber anderen Verfahren, die eine solche Erkennung nicht ermöglichen.

## Patentansprüche

1. Verfahren zum Ermitteln von Restflüssigkeit in einer rücklaufenden Mehrwegflasche (1), insbesondere einer PET-Flasche, wobei durch die Flaschenöffnung (4) in die Flasche Licht, vorzugsweise im Infrarotbereich, eingestrahlt wird und ausserhalb der Flasche (1) mindestens ein Teil des eingestrahlten Lichtes empfangen wird, und wobei das Empfangssignal zur Erkennung des Vorhandenseins von Restflüssigkeit ausgewertet wird, dadurch gekennzeichnet, dass der Empfänger Licht empfängt, welches an einer Wölbung (5) des Flaschenbodens reflektiert worden ist und seitlich aus der Flasche austritt.

2. Verfahren nach Anspruch 1, dadurch gekennzeichnet, dass das Empfangssignal zur Auswertung mit einem vorgewählten Grenzwert (18) verglichen wird.

3. Vorrichtung zum Ermitteln von Restflüssigkeit in einer rücklaufenden Mehrwegflasche (1), insbesondere einer PET-Flasche, bei welcher in die Flasche Licht, vorzugsweise im Infrarotbereich, eingestrahlt wird und ausserhalb der Flasche (1) mindestens ein Teil des eingestrahlten Lichtes empfangen wird, und bei welcher das Empfangssignal zur Erkennung des Vorhandenseins von Restflüssigkeit ausgewertet wird, umfassend mindestens eine Lichtquelle (3), vorzugsweise eine Infrarotlichtquelle, welche an einer Flaschenfördereinrichtung derart angeordnet ist, dass jeweils in mindestens eine Flasche (1) durch die Flaschenöffnung (4) Licht einstrahlbar ist, mindestens eine Empfangseinrichtung (6), welche an der Flaschenfördereinrichtung derart angeordnet ist, dass das aus der mindestens einen Flasche (1) austretende Licht empfangbar ist, und eine Auswerteinrichtung (9) zur Auswertung des Signals aus der Empfangseinrichtung (6), dadurch gekennzeichnet, dass die Empfangseinrichtung seitlich neben dem Bodenbereich der Flasche (1) angeordnet ist, derart, dass Licht empfangen wird, welches an einer Wölbung (5) des Flaschenbodens reflektiert worden ist.

4. Vorrichtung nach Anspruch 3, dadurch gekennzeichnet, dass die Empfangseinrichtung (6) eine Fokussieroptik (7) aufweist.

5. Vorrichtung nach einem der Ansprüche 3 oder 4, dadurch gekennzeichnet, dass die Lichtquelle (3) mindestens eine Infrarotleuchtdiode aufweist.

## Claims

1. Method for detecting residual liquid in a returned reusable bottle (1), in particular a PET bottle, in which light, preferably in the infrared range, is projected into the bottle through the bottle mouth (4) and at least part of the projected light is received outside the bottle (1), and in which the received signal is analysed so as to detect the presence of residual liquid, characterized in that the receiver receives light which has been reflected on a bulb (5) in the bottom of the bottle and emerges from the bottle laterally.

2. Method according to Claim 1, characterized in that the received signal is evaluated by comparison with a preselected limit value (18).

3. Apparatus for detecting residual liquid in a returned reusable bottle (1), in particular a PET bottle, in which light, preferably in the infrared range, is projected into the bottle and at least part of the projected light is received outside the bottle (1), and in which the received signal is analysed so as to detect the presence of residual liquid, comprising at least one light source (3), preferably an infrared light source, arranged on a bottle conveyor in such a way that light can be projected through the bottle mouth or mouths (4) into at least one bottle (1) at a time, at least one receiver device (6), arranged on the bottle conveyor so as to be capable of receiving the light emerging from the said bottle or bottles (1), and an evaluation unit (9) for evaluating the signal from the receiver device (6), characterized in that the receiver device is laterally located near the bottom end of the bottle (1) so as to receive light which has been reflected on a bulb (5) in the bottom of the bottle.

4. Apparatus according to Claim 3, characterized in that the receiver device (6) has a focusing lens (7).

5. Apparatus according to Claim 3 or Claim 4, characterized in that the light source (3) has at least one infrared light diode.

## Revendications

1. Procédé pour déterminer la présence d'un liquide résiduel dans une bouteille (1) réutilisable retournée, notamment une bouteille en polyéthylène, dans lequel de la lumière, de préférence de la lumière infra-rouge est introduite à travers l'ouverture (4) de la bouteille et dans lequel au moins une partie de la lumière introduite est détectée à l'extérieur de la bouteille (1), le signal de détection étant exploité pour la reconnaissance de la présence de liquide résiduel, caractérisé en ce que le détecteur reçoit de la lumière qui est réfléchie par une partie bombée (5) du fond de la bouteille et émerge latéralement de la bouteille.

2. Procédé selon la revendication 1, caractérisé en ce que pour l'exploitation, le signal de détection est comparé à une valeur limite (18) choisie d'avance.

3. Dispositif pour déterminer la présence d'un liquide résiduel se trouvant dans une bouteille (1) réutilisable retournée, notamment une bouteille en polyéthylène, dans lequel de la lumière, de préférence de la lumière infrarouge est introduite dans la bouteille, dans lequel au moins une partie de la lumière introduite est détectée à l'extérieur de la bouteille (1), et dans lequel le signal de détection est exploité pour la reconnaissance de la présence d'un liquide résiduel, Le dispositif comprenant au moins une source (3) de lumière, de préférence une source de lumière infrarouge, qui est agencée sur un dispositif transporteur de bouteilles de telle façon que de la lumière peut être introduite respectivement dans au moins une bouteille (1) à travers l'ouverture (4) de la bouteille, au moins un dispositif (6) de détection qui est agencé sur le dispositif transporteur de bouteilles de telle façon que la lumière émergeant de ladite au moins une bouteille (1) peut être détectée, et un dispositif (9) d'exploitation du signal délivré par le dispositif de détection (6), caractérisé en ce que le dispositif de détection est agencé latéralement, à côté de la zone du fond de la bouteille (1) de façon à détecter la lumière qui avait été réfléchie par une partie bombée (5) du fond de la bouteille.

4. Dispositif selon la revendication 3, caractérisé en ce que le dispositif de détection (6) présente un bloc-optique (7) de focalisation.

5. Dispositif selon la revendication 3 ou 4, caractérisé en ce que la source de lumière (3) présente au moins une diode luminescente émettant la lumière infra-rouge.
